# EUROPEAN PATENT SPECIFICATION

(11) **EP 4 454 582 B1**
(45) Date of publication and mention of the grant of the patent: **10.12.2025**
(21) Application number: 24172062.2
(22) Date of filing: 24.04.2024
(51) Int. Cl.: A61B 17/80

(54) **BONE PLATE FOR SECURING A FRACTURED BONE**
KNOCHENPLATTE ZUR SICHERUNG EINES GEBROCHENEN KNOCHENS
PLAQUE OSSEUSE POUR FIXER UN OS FRACTURÉ

(30) Priority: 27.04.2023 US 202363462290 P
(43) Date of publication of application: 30.10.2024
(73) Proprietor: Stryker European Operations Limited, Carrigtwohill, Co. Cork T45 HX08 (IE)
(72) Inventor: KEIL, Isabel, 79111 Freiburg (DE); SYLVESTRE, Pierre-Luc, 2540 Grenchen (CH)
(74) Representative: Röthinger, Rainer

(56) References cited:
- WO-A1-2010/014701
- US-A1- 2016 317 205
- US-A1- 2017 265 915
- US-A1- 2020 237 418

## Description

### BACKGROUND

The present disclosure relates to an implant permitting different pairs of screws layered in different trajectories for securing a fractured bone to pass therethrough. While discussed largely in connection with use in humeral bone plates, the present disclosure is applicable to other types of medical implants as well.

Fractures to bones are often the result of traumatic injuries. It is well known in the orthopedic arts to reduce and fix a bone fracture with a bone plate. For instance, fractures of the proximal humerus are often treated using bone plates specifically designed for such an application. Especially in older patients, the proximal humerus often has poor bone quality and does not support good screw purchase. Thus, the fixation of the proximal humerus using bone plates is difficult because the head of the humerus tends to collapse over the screws, causing damage to the joint and potential backing out of bone screws placed through the plate.

Current plate and screw designs are not optimal for good screw purchase in the proximal humerus, as well as other similar bones. This often results in less-than-ideal healing for the fractured bone. Thus, it would be desirable for a plate design which permits the placement of screws in a manner that reduces the relative motion of the bone along the screws for higher resistance to pull-out of screws and for higher resistance to bone collapse.

US 2020/237418 A1 discusses an anchor trajectory guide. The guide includes a body that has a medial side. The medial side can be placed over a lateral side of a fixation plate. The anchor trajectory guide also includes a locator and a plurality of guide apertures. The locator is disposed on or through the medial side of the body. The locator can be mated with the fixation plate. The guide apertures are disposed through the body at positions corresponding to define anchor locations and orientations to provide good purchase adjacent to or in cortical hone around a medial side of a humerus. WO 2010/014701 A1 discusses A periarticular bone plate for the internal fixation of a fractured, proximal tibia of a surgical patient includes a shaft member connected to a head member, a top surface, a bottom surface, a posterior edge, an anterior or edge and a plurality of holes extending between the top and bottom surfaces for receiving fasteners for attaching the plate to the bone. The head member is offset relative to the shaft member in each of a frontal and sagittal planes of the proximal tibia..

### BRIEF SUMMARY

A bone plate for securing a fractured bone is defined by independent claim 1, to which the reader should now refer. Specific embodiments are defined by dependent claims 2 to 15. The specific examples herein relate to apparatus and methods for securing a fractured bone with a bone plate to increase screw resistance to pull-out and resistance to bone collapse. These associated methods are also described herein to aid understanding the invention. These methods do not form part of the claimed invention. For instance, a bone plate in accordance with the present invention has at least four sets of screw holes that permit placement of screws in convergence, standard, convergence, and calcar screw trajectories from the top to the bottom ends of the plate. The orientation of the screw trajectories in the plate reduces the relative motion of the bone along the screws, thereby increasing resistance to pull-out of screws and resistance to bone collapse. The particular hole structures included in the bone plate permit insertion of the screws at various angles, thereby permitting the particular screw orientations discussed herein. This placement may be facilitated through the use of drill guides, screw insertion guides or the like.

In one embodiment, a bone plate for securing a fractured bone comprises a shaft portion and a head portion extending from the shaft portion along a longitudinal axis of the bone plate and including a plurality of holes. The plurality of holes comprises a first pair of screw holes including a first hole and a second hole having central axes that converge towards each other, a second pair of screw holes including a third hole and a fourth hole having central axes that are substantially parallel to each other, a third pair of screw holes including a fifth hole and a sixth hole having central axes that converge towards each other, and a fourth pair of screw holes including a seventh hole and an eighth hole. The seventh and eighth holes have central axes angled towards a most proximal end of the head portion.

In some examples, the first pair of screw holes are positioned between the tip of the head portion and at least one of the second, third, or fourth pairs of screw holes. In some examples, the second pair of screw holes are positioned between the first pair of screw holes and the shaft portion. In some examples, the third pair of screw holes are positioned between the second pair of screw holes and the shaft portion. In some examples, the fourth pair of screw holes are positioned between the third pair of screw holes and the shaft portion.

In some examples, the fourth pair of screw holes are configured to align with a calcar region of bone.

In some examples, the entire head portion is wider than the shaft portion in a direction perpendicular to the longitudinal axis along an upper surface of the plate.

In some examples, the bone plate comprising threads within at least one hole of the plurality of holes, and the threads configured to engage a head of a bone screw. In some examples, the threads extend only partially through the at least one hole.

In some examples, the plurality of screw holes further includes a ninth hole disposed between the first and second holes.

In some examples, the plurality of screw holes further includes a tenth hole disposed adjacent and forming a triangle with the seventh and eighth holes, and the tenth hole converges toward the seventh and eighth holes beneath a lower surface of the bone plate.

In some examples, at least one of the first, second, third, or fourth pairs of screw holes are disposed on opposite sides of the longitudinal axis of the bone plate.

In some examples, the first and second holes are asymmetrically disposed on the opposite sides of the longitudinal axis.

In some examples, at least one hole of the plurality of holes includes a plurality of spaced apart scalloped regions.

In some examples, the third and fourth central hole axes do not converge beneath a lower surface of the bone plate.

Another example of the present invention is a method (not claimed) of securing a bone fracture including the steps of placing a lower surface of a bone plate against a bone, driving a first screw and a second screw through a proximal end of the bone plate along converging trajectories, driving a third screw and a fourth screw through the bone plate at a location distal of the first and second screws, driving a fifth screw and a sixth screw through the bone plate at a location distal of the third and fourth screws so that the fifth and sixth screws extend along converging trajectories, and driving a seventh and an eighth screw through the bone plate at a location distal of the fifth and sixth screws.

Again, the foregoing example may vary in other embodiments. For instance, the method may include driving the first and second screws, the third and fourth screws, the fifth and sixth screws, or the seventh and eighth screws through their respective screw holes, which may be on opposite sides of the longitudinal axis of the plate. In another embodiment, the method may include driving the third and fourth screws along substantially parallel trajectories. The method may include driving a portion of the seventh and eighth screws into a calcar region of the humerus. The method includes guiding the bone plate along a k-wire to contact the bone.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIGs. 1A, B, and C are perspective and side views of a bone plate in accordance with an embodiment of the present invention implanted on a bone with a first set of screws extending therethrough;
FIGs. 2 A, B, and C are perspective and side views of the plate of FIG. 1A implanted on the bone with a second set of screws extending therethrough;
FIGs. 3 A, B, and C are perspective and side views of the plate of FIG. 1A implanted on the bone with a third set of screws extending therethrough;
FIGs. 4 A, B, and C are perspective and side views of the plate of FIG. 1A implanted on the bone with a fourth set of screws extending therethrough;
FIG. 5 is a side view of the implanted plate of FIG. 1A;
FIG. 6 is a top view of the implanted plate of FIG. 1A;
FIGs. 7 A and B are perspective and side views of the bone plate of FIG. 1A without screws extending therethrough;
FIG. 8 is a top view of the bone plate of FIG. 1A without screws extending therethrough; and
FIG. 9 is a cross-sectional anterior-posterior view of the bone plate of FIG. 1A taken in the mid-plane of K-wire slot 16 without screws extending therethrough.

### DETAILED DESCRIPTION

As used herein unless stated otherwise, the term "anterior" means toward the front part of the body, and the term "posterior" means toward the back part of the body. When referring to specific directions in the following description, the terms "proximal" and "distal" are to be understood in regard to the device's orientation and position during exemplary application to human body. Thus, the term "proximal" means closer to the operator or in a direction toward the operator, and the term "distal" means more distant from the operator or in a direction away from the operator. In addition, the terms "about," "generally," and "substantially" are intended to mean that deviations from absolute are included within the scope of the term so modified.

Turning now to the figures, a bone plate is shown and will be discussed below. The particular examples shown permit the fixation of a bone plate to the bone using at least four pairs of screws at different orientations. However, the various examples of the bone plate disclosed below are not limited to just the specific designs and uses disclosed herein. For instance, while shown generally as a humeral bone plate, the present invention has applicability for other types of bone plates (*e.g.,* for other long bones, craniomaxillofacial, small fragments, or the like).

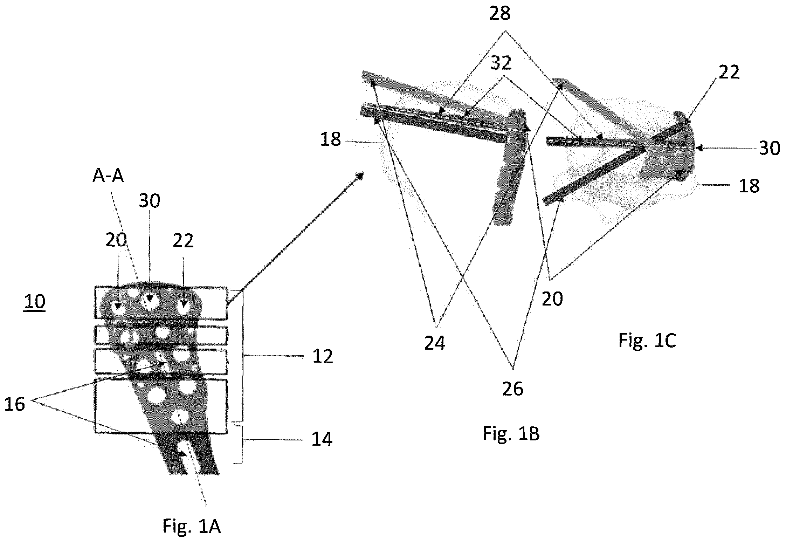

As shown in FIG. 1A, in accordance with one embodiment of the present invention, a bone plate 10 includes a head portion 12, a shaft portion 14, multiple slots 16, 17, and a first pair of screw holes comprising first screw hole 20 and second screw hole 22. Head portion 12 is located on the proximal end of plate 10, while shaft portion 14 is located on the distal end of plate 10. Head portion 12 is wider than shaft portion 14. Slot 16 is located towards the middle of head portion 12 and towards the middle of shaft portion 14 on plate 10, and is designed to accommodate a K-wire or the like. Slot 17 is, on the hand, designed to accept a bone screw and may be a compression slot or the like. First screw hole 20 and second screw hole 22 are located on opposite sides of the head portion 12 and are asymmetrically disposed with respect to a longitudinal axis running from the anterior to posterior ends of plate 10. A ninth screw hole 30 is disposed between first screw hole 20 and second screw hole 22. Holes 20, 22, and 30 are not linearly aligned, as shown in the drawings. Moreover, the specific configurations of slots 16, 17 can vary depending upon the type of implant/plate being utilized.

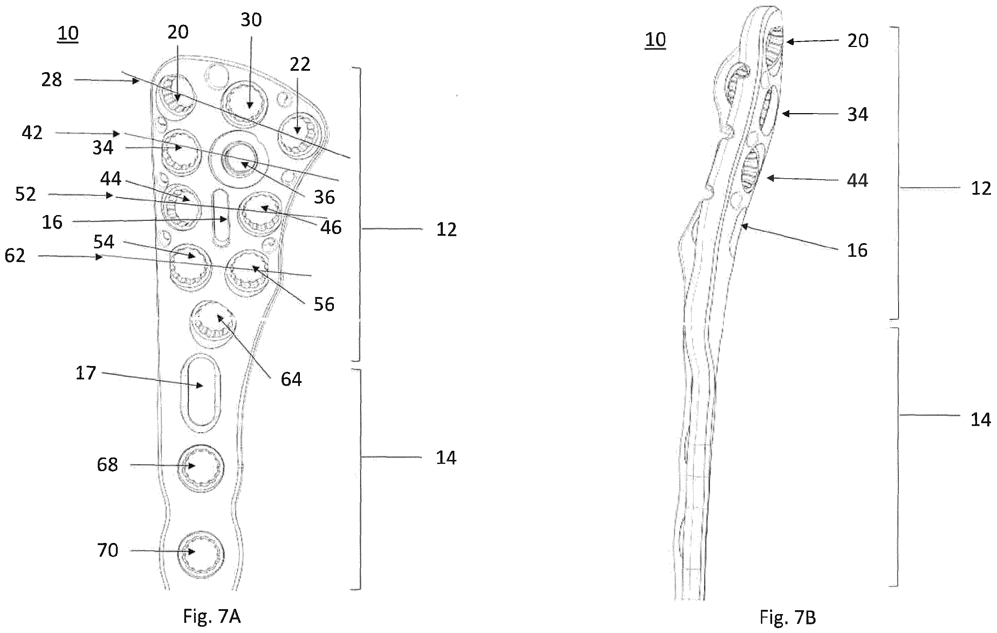

FIGs. 1B and C depict the bone plate of FIG. 1A attached to bone 18. First screw 24 and second screw 26 pass through first screw hole 20 and second screw hole 22, respectively, and converge towards each other. First screw 24 is representative of the first central hole axis, and second screw 26 is representative of the second central hole axis. A first imaginary line 28 passes through the first screw hole 20 and second screw hole 22, as is best shown in FIG. 7A.

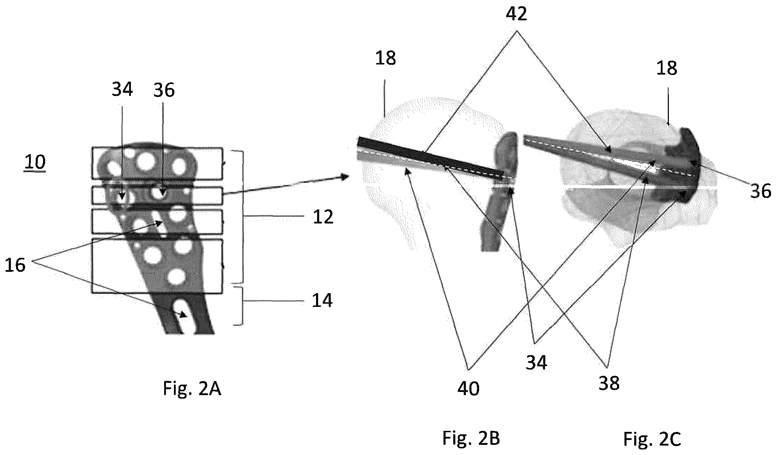

FIG. 2A again depicts the bone plate 10 of FIG. 1A, but with a focus on a second pair of screw holes comprising third screw hole 34 and fourth screw hole 36, which are located on opposite sides of the head portion 12. FIGs. 2B and C depict the bone plate of FIG. 1A attached to bone 18, with third screw 38 and fourth screw 40 passing through third screw hole 34 and fourth screw hole 36, respectively. As shown, these screws are substantially parallel and do not converge towards each other. Third screw 38 is representative of the third central hole axis, and fourth screw 40 is representative of the fourth central hole axis. A second imaginary line 42 passes through third screw hole 34 and fourth screw hole 36, as is best shown in FIG. 7A.

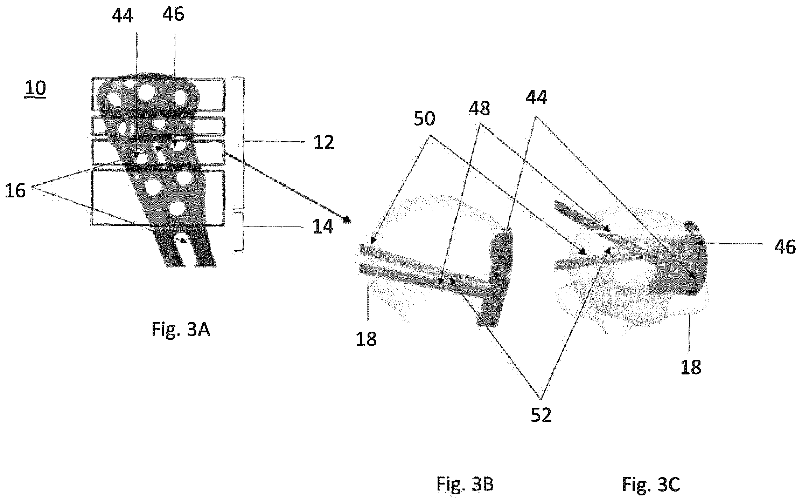

FIG. 3A depicts the bone plate 10 of FIG. 1A, but with a focus on a third pair of screw holes comprising fifth screw hole 44 and sixth screw hole 46, which are located on opposite sides of the head portion 12. FIGs. 3B and C depict the bone plate of FIG. 1A attached to bone 18, with fifth screw 48 and sixth screw 50 passing through fifth screw hole 44 and sixth screw hole 46, respectively, and converging towards each other. Fifth screw 48 is representative of the fifth central hole axis, and sixth screw 50 is representative of the sixth central hole axis. A third imaginary line 52 passes through fifth screw hole 44 and sixth screw hole 46, as is best shown in FIG. 7A.

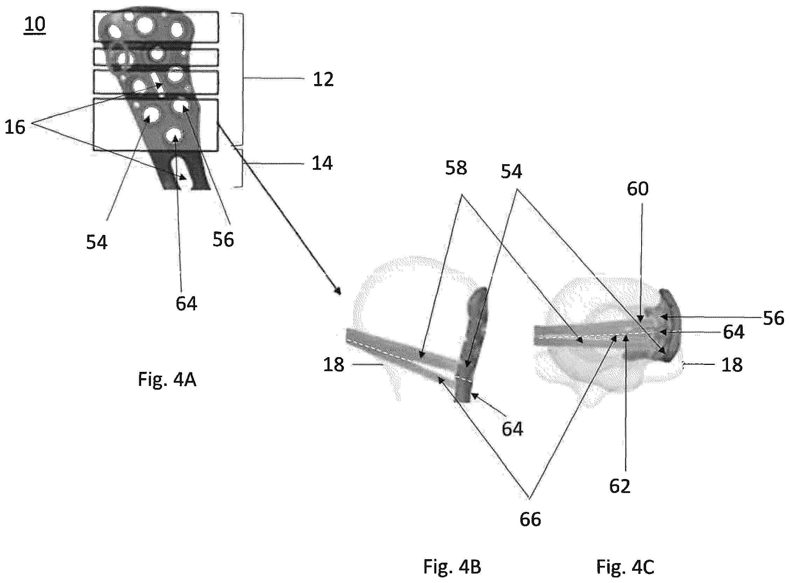

FIG. 4A depicts the bone plate 10 of FIG. 1A, but with a focus on a fourth pair of screw holes comprising seventh screw hole 54 and eighth screw hole 56, which are located on opposite sides of the head portion 12. Bone plate 10 further includes a tenth screw hole 64 disposed adjacent and forming a triangle with seventh screw hole 54 and eighth screw hole 56. Tenth screw 66 passes through tenth screw hole 64. FIGs. 4B and C depict the bone plate of FIG. 1A attached to bone 18, with seventh screw 58 and eighth screw 60 passing through seventh screw hole 54 and eighth screw hole 56, respectively. As shown, these screws are angled upward towards a most proximal end of the head portion, configured to be aligned with a calcar region of the bone, and do not converge towards each other. Tenth screw 66 converges toward seventh screw 58 and eighth screw 60. Seventh screw 58 is representative of the seventh central hole axis, eighth screw 60 is representative of the eighth central hole axis, and tenth screw 66 is representative of the tenth central hole axis. A fourth imaginary line 62 passes through seventh screw hole 54 and eighth screw hole 56, as is best shown in FIG. 7A.

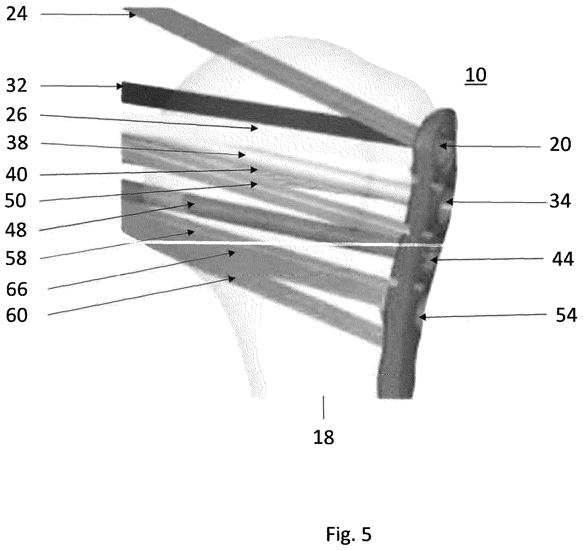

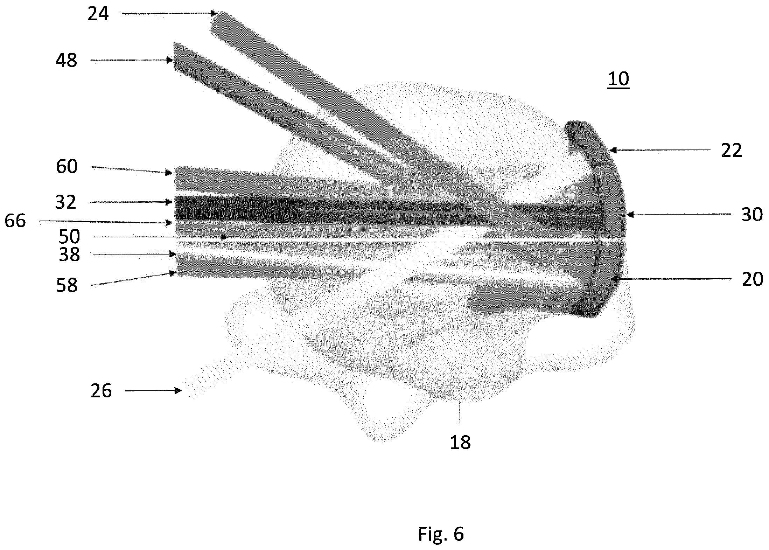

FIG. 5 is a side view of the bone plate 10 of FIG. 1A attached to bone 18, while FIG. 6 is a top view of the bone plate 10 of FIG. 1A attached to bone 18. Both figures depict the screw placement discussed above by including the same screws shown passing through the bone. As previously described, plate 10 has a first screw 24 and second screw 26 passing through first screw hole 20 and second screw hole 22, respectively; a third screw 38 and fourth screw 40 passing through third screw hole 34 and fourth screw hole 36, respectively; a fifth screw 48 and sixth screw hole 50 passing through fifth screw hole 44 and sixth screw hole 46, respectively; and a seventh screw 58 and eighth screw 60 passing through seventh screw hole 54 and eighth screw hole 56, respectively. In some embodiments, ninth screw 32 passes through ninth screw hole 30, and tenth screw 66 passes through tenth screw hole 64. These screws are oriented in the manners discussed above to improve upon the fixation of plate 10 to bone 18.

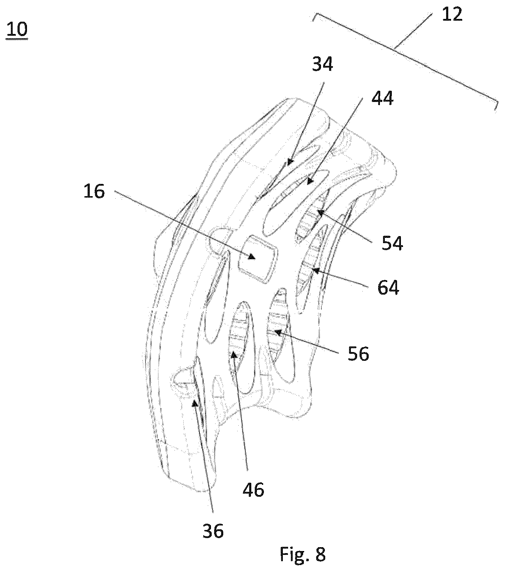

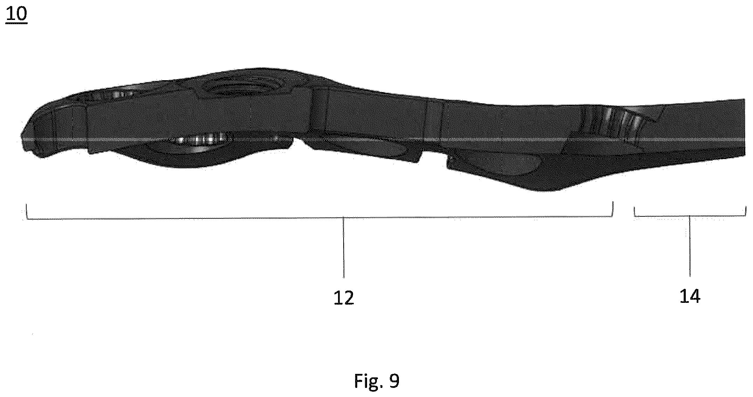

FIGs. 7A and B, 8 and 9 are perspective, side, top, and cross-section views of bone plate 10 of FIG. 1A, respectively, without screws extending therethrough. As shown in FIG. 7A, there may be an eleventh screw hole 68 and a twelfth screw hole 70 on the shaft portion 14 on the distal end of plate 10.

As previously described, a first imaginary line 28 passes through the first screw 24 and second screw 26; a second imaginary line 42 passes through the third screw 38 and fourth screw 40; a third imaginary line 52 passes through the fifth screw 48 and sixth screw 50; and a fourth imaginary line 62 passes through the seventh screw 58 and eighth screw 60. First imaginary line 28, second imaginary line 42, third imaginary line 52, and fourth imaginary line 62 are sequentially disposed from the proximal to distal end of plate 10. First imaginary line 28 is closest to the proximal end of plate 10, whereas fourth imaginary line 62 is closest to the distal end. The orientation of the screws along the imaginary lines improves bone fixation because the screws are at different height levels along most of the humerus head, therefore preventing the bone from collapsing away or towards the plate.

In accordance with the present invention, the convergence, standard, convergence, and calcar screw trajectories can reduce the relative motion of the bone along the screws. These trajectories therefore can result in a higher resistance to pull-out of screws or to bone collapse. In the prior art, screws are parallel to each other, so the bone is prone to collapsing since the mechanical resistance is in one direction. The orientation of the screws in both converging and diverging trajectories prevents bone collapse because of the mechanical resistance in differing directions. Additionally, the threading of the screws provides further support to the bone to prevent bone collapse.

In the embodiments shown, the screw holes are variable angle holes, which permits screws to be placed at various angles therethrough. For example, the variable angle holes may be holes like those disclosed in U.S. Patent Application Serial No. 16/289,826 (now U.S. Patent No. 11,039,865). Of course, in other embodiments, different variable angle hole designs may be employed. In other embodiments, the screw holes can be single axis holes or can include threading. These non-variable holes would include hole axes aligned to require placement of the screws in accordance with the foregoing discussion. For instance, threaded holes would have fixed angle axes aligned in the manners disclosed above. In alternative embodiments, the screw holes may include additional fixation features, such as scallops, or the like.

Bone plate 10, and any other implant in accordance with the present invention, can be made of any material suitable for implanting into a human body, including but not limited to polymer materials (*e.g.,* PEEK) and metallic materials (*e.g.,* stainless steel or titanium). Plates according to the present invention can be manufactured utilizing any known process, including but not limited to additive manufacturing or the like. It is also contemplated to form certain portions of plate 10 via one process and others via another process. For instance, plate 10 can be 3D printed, with the plate holes thereafter being formed in the plate.

A method (not claimed) of securing a bone fracture using a plate according to the present invention will now be described. The method generally includes placing the lower surface of plate 10 against a bone. This can be done using any means of guiding the plate 10, such as through the use of one or more k-wires. Before the screws are inserted, a threaded hole in the plate may be used for engaging a guide and the bores in the guide may dictate the path of the screws to be inserted. For example, screw hole 36 can be a threaded hole for the guide. Screws may be inserted into slot 17 to allow for plate adjustment before the plate is fixed into place with the remaining screws.

First screw 24 and second screw 26 are then driven through first screw hole 20 and second screw hole 22, respectively, from an upper surface to the lower surface of the bone plate and at the proximal end of plate 18 along converging trajectories. Third screw 38 and fourth screw 40 are then driven through third screw hole 34 and fourth screw hole 36, respectively, at a location distal of first screw 24 and second screw 26 along substantially parallel trajectories. Fifth screw 44 and sixth screw 46 are then driven through fifth screw hole 48 and sixth screw hole 50, respectively at a location distal of third screw 38 and fourth screw 40 along converging trajectories. Seventh screw 54 and eighth screw 56 are then driven through seventh screw hole 58 and eighth screw hole 60, respectively, at a location distal of fifth screw 44 and sixth screw 46 in calcar trajectories. Ninth screw 32 and tenth screw 66 are also driven through ninth screw hole 30 and tenth screw hole 64, respectively.

It is to be understood that this method may differ based on surgeon preference, location of the fracture lines, or the needs of the patient. Specifically, the order of insertion may differ for first screw 24, second screw 26, third screw 38, fourth screw 40, fifth screw 44, sixth screw 46, seventh screw 54, eighth screw 56, ninth screw 32, and tenth screw 66 in their respective screw holes.

The foregoing methodology may involve the use of standard tools, such as screw drivers and drills to prepare the bone and place the screws. Guides, such as drill and screw placement guides, can also be utilized. This is particularly true in connection with embodiment plates having variable angle screw holes. The use of guides can dictate the placement of the screws according to the orientations described above. For instance, such guides may force the screws into the convergence, standard, convergence, and calcar screw trajectories noted above. It is also contemplated to utilize a plate according to the present invention along with an intramedullary nail, such that screws pass through both devices.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature can also be used, to the extent possible, in combination with and/or in the context of other particular aspects, arrangements, configurations, and arrangements of the technology, and in the technology generally.

Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative arrangements and that other arrangements may be devised without departing from the scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A bone plate (10) for securing a fractured bone, the bone plate (10) comprising:
a shaft portion (14); and
a head portion (12) extending from the shaft portion (14) along a longitudinal axis of the bone plate (10) and including a plurality of holes comprising:
a first pair of screw holes including a first hole (20) and a second hole (22) having central axes that converge towards each other;
a second pair of screw holes including a third hole (34) and a fourth (36) hole having central axes that are parallel to each other:
a third pair of screw holes including a fifth hole (44) and a sixth hole (46) having central axes that converge towards each other; and
a fourth pair of screw holes including a seventh hole (54) and an eighth hole (56), the seventh (54) and eighth holes (56) having central axes angled towards a most proximal end of the head portion (12).

2. The bone plate (10) of claim 1, wherein the first pair of screw holes (20, 22) are positioned between the tip of the head portion (12) and at least one of the second (34, 36), third (44, 46), or fourth (54, 56) pairs of screw holes.

3. The bone plate (10) of claim 1 or 2, wherein the second pair of screw holes (34, 36) are positioned between the first pair of screw holes (20, 22) and the shaft portion (14).

4. The bone plate (10) of any of the preceding claims, wherein the third pair of screw holes (44, 46) are positioned between the second pair of screw holes (34, 36) and the shaft portion (14).

5. The bone plate (10) of any of the preceding claims, wherein the fourth pair of screw holes (54, 56) are positioned between the third pair of screw holes (44, 46) and the shaft portion (14).

6. The bone plate (10) of any of the preceding claims, wherein the fourth pair of screw holes (54, 56) are configured to align with a calcar region of bone.

7. The bone plate (10) of any of the preceding claims, wherein the entire head portion (12) is wider than the shaft portion (14) in a direction perpendicular to the longitudinal axis along an upper surface of the plate (10).

8. The bone plate (10) of any of the preceding claims, further comprising threads within at least one hole of the plurality of holes, the threads configured to engage a head of a bone screw.

9. The bone plate (10) of claim 8, wherein the threads extend only partially through the at least one hole.

10. The bone plate (10) of any of the preceding claims, wherein the plurality of screw holes further includes a ninth hole (30) disposed between the first (20) and second holes (22).

11. The bone plate (10) of any of the preceding claims, wherein the plurality of screw holes further includes a tenth hole (64) disposed adjacent and forming a triangle with the seventh (54) and eighth (56) holes, wherein the tenth hole (64) converges toward the seventh (54) and eighth (56) holes beneath a lower surface of the bone plate (10).

12. The bone plate (10) of any of the preceding claims, wherein at least one of the first (20, 22), second (34, 36), third, (44, 46), or fourth pairs of screw holes (54, 56) are disposed on opposite sides of the longitudinal axis of the bone plate (10).

13. The bone plate (10) of any of the preceding claims, wherein the first (20) and second (22) holes are asymmetrically disposed on the opposite sides of the longitudinal axis.

14. The bone plate (10) of any of the preceding claims, wherein at least one hole of the plurality of holes includes a plurality of spaced apart scalloped regions.

15. The bone plate (10) of any of the preceding claims, wherein the third and fourth central hole axes do not converge beneath a lower surface of the bone plate (10).

## Patentansprüche

1. Knochenplatte (10), um einen gebrochenen Knochen zu fixieren, wobei die Knochenplatte (10) umfasst:
einen Schaftabschnitt (14); und
einen Kopfabschnitt (12), welcher sich vom Schaftabschnitt (14) ausgehend entlang einer Längsachse der Knochenplatte (10) erstreckt und eine Vielzahl an Löchern aufweist, die umfassen:
ein erstes Paar Schraubenlöcher, einschließlich eines ersten Loches (20) und eines zweiten Loches (22), welche Mittelachsen haben, die aufeinander zu konvergieren;
ein zweites Paar Schraubenlöcher, einschließlich eines dritten Loches (34) und eines vierten Loches (36), welche zueinander parallele Mittelachsen haben;
ein drittes Paar Schraubenlöcher, einschließlich eines fünften Loches (44) und eines sechsten Loches (46), welche Mittelachsen haben, die aufeinander zu konvergieren; und
ein viertes Paar Schraubenlöcher, einschließlich eines siebten Loches (54) und eines achten Loches (56), wobei das siebte (54) und achte Loch (56) Mittelachsen haben, die zu einem proximalsten Endes des Kopfabschnitts (12) hin geneigt sind.

2. Knochenplatte (10) nach Anspruch 1, wobei das erste Paar Schraubenlöcher (20, 22) zwischen der Spitze des Kopfabschnittes und mindestens einem der zweiten (34, 36), dritten (44,46) oder vierten (54, 56) Paare Schraubenlöcher liegt.

3. Knochenplatte (10) nach Anspruch 1 oder 2, wobei das zweite Paar Schraubenlöcher (34, 36) zwischen dem ersten Paar Schraubenlöcher (20, 22) und dem Schaftabschnitt (14) positioniert ist.

4. Knochenplatte (10) nach einem vorhergehenden Anspruch, wobei das dritte Paar Schraubenlöcher (44, 46) zwischen dem zweiten Paar Schraubenlöcher (34, 36) und dem Schaftabschnitt (14) positioniert ist.

5. Knochenplatte (10) nach einem vorhergehenden Anspruch, wobei das vierte Paar Schraubenlöcher (54, 56) zwischen dem dritten Paar Schraubenlöcher (44, 46) und dem Schaftabschnitt (14) positioniert ist.

6. Knochenplatte (10) nach einem vorhergehenden Anspruch, wobei das vierte Paar Schraubenlöcher (54, 56) auf eine Weise konfiguriert ist, dass es in Bezug auf eine Calcar-Region des Knochens ausgerichtet ist.

7. Knochenplatte (10) nach einem vorhergehenden Anspruch, wobei der gesamte Kopfabschnitt (12) breiter ist als der Schaftabschnitt (14) in einer senkrechten Richtung zu der Längsachse entlang der Oberseite der Platte (10).

8. Knochenplatte (10) nach einem vorhergehenden Anspruch, weiter umfassend ein Gewinde durch mindestens eines der Vielzahl an Löchern, wobei das Gewinde dazu konfiguriert ist, mit dem Kopf einer Knochenschraube zusammenzuwirken.

9. Knochenplatte (10) nach Anspruch 8, wobei sich das Gewinde nur teilweise durch mindestens eines der Löcher erstreckt.

10. Knochenplatte (10) nach einem vorhergehenden Anspruch, wobei die Vielzahl der Schraubenlöcher außerdem ein neuntes Schraubenloch (30) enthält, welches zwischen dem ersten (20) und zweiten Schraubenloch (22) angeordnet ist.

11. Knochenplatte (10) nach einem vorhergehenden Anspruch, wobei die Vielzahl der Schraubenlöcher außerdem ein zehntes Schraubenloch (64) enthält, welches neben dem siebten (54) und achten (56) Schraubenloch angeordnet ist und mit dem siebten (54) und achten (56) Schraubenloch ein Dreieck bildet, wobei das zehnte Schraubenloch (64) unterhalb einer Unterseite der Knochenplatte (10) zum siebten (54) und achten (54) Schraubenloch hin konvergiert.

12. Knochenplatte (10) nach einem vorhergehenden Anspruch, wobei mindestens eines der ersten (20, 22), zweiten (34, 36), dritten (44, 46) oder vierten (54, 56) Paare Schraubenlöcher auf gegenüberliegenden Seiten der Längsachse der Knochenplatte (10) liegt.

13. Knochenplatte (10) nach einem vorhergehenden Anspruch, wobei das erste (20) und zweite (22) Loch zueinander asymmetrisch auf gegenüberliegenden Seiten der Längsachse liegen.

14. Knochenplatte (10) nach einem vorhergehenden Anspruch, wobei mindestens eines der Vielzahl an Löchern eine Vielzahl an voneinander getrennten, bogenförmigen Regionen haben.

15. Knochenplatte (10) nach einem vorgehenden Anspruch, wobei die dritte und vierte Zentrallochachse unterhalb einer Unterseite der Knochenplatte (10) nicht konvergieren.

## Revendications

1. Plaque osseuse (10) destinée à fixer un os fracturé, la plaque osseuse (10) comprenant:
une partie tige (14); et
une partie tête (12) s'étendant depuis la partie tige (14) le long d'un axe longitudinal de la plaque osseuse (10) et comprenant une pluralité de trous comprenant:
une première paire de trous de vis comprenant un premier trou (20) et un deuxième trou (22) dont les axes centraux convergent l'un vers l'autre;
une deuxième paire de trous de vis comprenant un troisième trou (34) et un quatrième trou (36) dont les axes centraux qui parallèles entre eux;
une troisième paire de trous de vis comprenant un cinquième trou (44) et un sixième trou (46) dont les axes centraux convergent l'un vers l'autre; et
une quatrième paire de trous de vis comprenant un septième trou (54) et un huitième trou (56), les septième (54) et huitième (56) trous ayant des axes centraux inclinés vers une extrémité la plus proximale de la partie tête (12).

2. Plaque osseuse (10) selon la revendication 1, dans laquelle la première paire de trous de vis (20, 22) est positionnée entre l'extrémité de la partie tête (12) et au moins l'une parmi la deuxième (34, 36), la troisième (44, 46) ou quatrième (54, 56) paires de trous de vis.

3. Plaque osseuse (10) selon la revendication 1 ou 2, dans laquelle la deuxième paire de trous de vis (34, 36) est positionnée entre la première paire de trous de vis (20, 22) et la partie tige (14).

4. Plaque osseuse (10) selon l'une quelconque des revendications précédentes, dans laquelle la troisième paire de trous de vis (44, 46) est positionnée entre la deuxième paire de trous de vis (34, 36) et la partie tige (14).

5. Plaque osseuse (10) selon l'une quelconque des revendications précédentes, dans laquelle la quatrième paire de trous de vis (54, 56) est positionnée entre la troisième paire de trous de vis (44, 46) et la partie tige (14).

6. Plaque osseuse (10) selon l'une quelconque des revendications précédentes, dans laquelle la quatrième paire de trous de vis (54, 56) est conçue pour s'aligner avec une zone calcaire de l'os.

7. Plaque osseuse (10) selon l'une quelconque des revendications précédentes, dans laquelle la partie tête (12) entière est plus large que la partie tige (14) dans une direction perpendiculaire à l'axe longitudinal le long d'une face supérieure de la plaque (10).

8. Plaque osseuse (10) selon l'une quelconque des revendications précédentes, comprenant en outre des filetages dans au moins un trou parmi la pluralité de trous, les filetages étant conçus pour s'engrener dans une tête d'une vis à os.

9. Plaque osseuse (10) selon la revendication 8, dans laquelle les filetages ne s'étendent que partiellement à travers le ou les trous.

10. Plaque osseuse (10) selon l'une quelconque des revendications précédentes, dans laquelle la pluralité de trous de vis comprend en outre un neuvième trou (30) disposé entre les premier (20) et deuxième (22) trous.

11. Plaque osseuse (10) selon l'une quelconque des revendications précédentes, dans laquelle la pluralité de trous de vis comprend en outre un dixième trou (64) disposé à proximité d'un triangle et formant ce dernier avec les septième (54) et huitième (56) trous, le dixième trou (64) convergeant en direction des septième (54) et huitième (56) trous sous une face inférieure de la plaque osseuse (10).

12. Plaque osseuse (10) selon l'une quelconque des revendications précédentes, dans laquelle au moins l'une parmi les première (20, 22), deuxième (34, 36), troisième (44, 46) ou quatrième paires de trous de vis (54, 56) est disposée sur les côtés opposés de l'axe longitudinal de la plaque osseuse (10).

13. Plaque osseuse (10) selon l'une quelconque des revendications précédentes, dans laquelle les premier (20) et deuxième (22) trous sont disposés de manière asymétrique sur les côtés opposés de l'axe longitudinal.

14. Plaque osseuse (10) selon l'une quelconque des revendications précédentes, dans laquelle au moins un trou parmi la pluralité de trous comprend une pluralité de régions festonnées espacées les unes des autres.

15. Plaque osseuse (10) selon l'une quelconque des revendications précédentes, dans laquelle les troisième et quatrième axes centraux des trous ne convergent pas sous une face inférieure de la plaque osseuse (10).
